# FASCICULE DE BREVET EUROPEEN

(11) **EP 0 416 984 B1**
(45) Date de publication et mention de la délivrance du brevet: **16.06.1993**
(21) Numéro de dépôt: 90402399.1
(22) Date de dépôt: 30.08.1990
(51) Int. Cl.: G01N 33/564, G01N 33/68

(54) **Moyens pour le diagnostic de neuropathies démyélinisantes, en particulier de la sclérose en plaques**
Mittel zum Nachweis von Gehirnkrankheiten in Zusammenhang mit Nervenscheidenmarkzerstörung, insbesondere Sklerosis Multiplex
Means for the diagnosis of neurological demyelinisation disorders, especially multiple sclerosis

(30) Priorité: 06.09.1989 FR 8911667
(43) Date de publication de la demande: 13.03.1991
(73) Titulaire: CENTRE NATIONAL DE LA RECHERCHE SCIENTIFIQUE, 75007 Paris (FR)
(72) Inventeur: Zanetta, Jean-Pierre, F-67370 Griesheim S/Souffel (FR); Warter, Jean-Marie, F-67000 Strasbourg (FR); Kuchler, Sabine, F-67000 Strasbourg (FR); Vincendon, Guy, F-67000 Strasbourg (FR)
(74) Mandataire: Peaucelle, Chantal

(56) Documents cités:
- EP-A- 0 337 799
- WO-A-89/00581
- US-A- 4 311 686
- DEVELOPMENTAL NEUROSCIENCE, vol. 10, no. 3, 1988, BASEL (CH); S.KUCHLER et al., pp. 199-212*
- JOURNAL OF NEUROCHEMISTRY, vol. 49, 1987, NEW YORK, NY (US); J.P.ZANETTA et al., pp. 1250-1257*
- CLINICAL CHEMISTRY, vol. 27, no. 12, décembre 1981, WINSTON, (US); B.GERSON et al., pp. 1974-1977*
- LANCET, vol. 335, 1990, LONDON (GB); J-P.ZANETTA et al., pp. 1482-1484*

## Description

L'invention concerne des moyens pour le diagnostic in vitro de neuropathies démyélinisantes, en particulier de la sclérose en plaques ou SEP.

Les études statistiques de l'Organisation Mondiale de la Santé montrent que la SEP est une maladie en extension dans le monde et notamment dans ses régions de prédilection que sont les pays de climat tempéré et froid. 50.000 cas sont répertoriés en France, le nombre de cas réels devant être supérieur. La même proportion de malades se retrouve dans l'ensemble des pays d'Europe et d'Amérique du Nord à climat tempéré ou froid, ainsi que dans les zones correspondantes de l'hémisphère sud.

L'étude de la SEP a montré que des facteurs variés, génétiques et/ou géographiques, ou encore infectieux, peuvent jouer un rôle dans son déclenchement. La nature de ces agents et leur mécanisme d'action dans le déclenchement de la SEP ne sont pas élucidés à ce jour. Il est admis cependant qu'à un certain stade, la maladie évolue en une attaque autoimmune de la myéline dans les tissus du système nerveux central avec formation de plaques de myéline dégénérée.

On observe également une invasion de macrophages et de différents types de lymphocytes et un isolement final des plaques par des astrocytes.

Les analyses biochimiques effectuées n'ont pas permis d'identifier jusqu'à présent de constituants spécifiques de la myéline (que ce soit des lipides, des acides nucléiques ou des protéines), dotés de propriétés antigéniques et constituant la cible immunologique des anticorps produits dans cette maladie.

Après les échecs des expériences tentant de mettre en évidence dans les liquides céphalo-rachidiens de patients atteints de SEP des anticorps dirigés contre des antigènes protéiques ou lipidiques majeurs de la myéline, certains groupes de recherche se sont intéressés à la Myelin Associated Glycoprotein MAG puis à la Myelin-Oligodendrocyte Glycoprotein MOG, sans que des résultats positifs en soient clairement issus.

Une autre approche a consisté a rechercher si les anticorps présents dans les liquides céphalo-rachidiens de patients atteints de SEP étaient dirigés contre des épitopes viraux et en particulier des rétrovirus du type HTLV-1. Les résultats obtenus ont montré cependant une spécificité insuffisante de la réaction, de très nombreuses réactions positives ayant été observées chez des patients atteints d'autres maladies neurologiques que les neuropathies.

US-A-4311686 décrit le diagnostic de scléroses multiples et de maladies malignes pas analyse d'échantillon sanguin. La méthodologie décrite est basée sur l'isolement, à partir d'un pool de fluides biologiques provenant de donneurs atteints de scléroses multiples, d'une substance qui aurait des caractéristiques antigéniques spécifiques lorsqu'on la fait réagir avec des leucocytes sensibilisés à la maladie d'un échantillon de sang entier.

Developmental Neuroscience 1988, 10, 199-212 rapporte le rôle de la lectine cerebelleuse soluble dans l'adhésion cellulaire et la formation de myéline dans des oligodendrocytes de rat. Ce document n'enseigne pas que les C.S.L. sont reconnues spécifiquement par des anticorps produits par des neuropathies démyélinisantes.

EP-A-337799, qui fait partie de l'art antérieur de la technique selon l'Article 54.3 CBE, décrit une lectine ayant une affinité pour le β-D-galactoside, isolée à partir d'une lignée cellulaire humaine de leucémie, ou à partir de tissus de placenta humain. Il est envisagé d'utiliser ces lectines dans des applications thérapeutiques et diagnostiques. Leur utilisation dans le traitement de maladies auto-immunes, telles que la sclérose en plaques, est évoquée. La lectine est différente de celle de la présente invention.

L'étude par les inventeurs de lectines particulières liant les glycanes riches en mannose a montré qu'elles correspondent à des antigènes spécifiquement reconnus par des anticorps présents dans les liquides céphalo-rachidiens et le sang de malades atteints de neuropathies démyélinisantes, et plus particulièrement de sclérose en plaques.

L'invention vise donc l'utilisation de telles lectines pour le diagnostic in vitro de neuropathies démyélinisantes.

Elle a plus particulièrement pour but de fournir des moyens de diagnostic de ces maladies, à savoir une méthode permettant de les détecter à un stade précoce en utilisant lesdites lectines et un kit renfermant les éléments permettant d'effectuer le diagnostic.

L'invention est ainsi caractérisée par l'utilisation pour le diagnostic de neuropathies démyélinisantes, notamment de la SEP, de lectines reconnaissant les glycanes riches en mannose, ou de leurs sous-unités protéiques, ces lectines ou leurs sous-unités présentant une parenté immunologique respectivement avec les lectines cerebelleuses solubles "cerebellar soluble lectin" ou "CSL" ou leurs sous-unités protéiques.

L'expression "parenté immunologique" signifie que ces lectines endogènes, ou leurs sous-unités, sont capables de former des complexes du type antigène-anticorps avec les anticorps reconnaissant respectivement les CSL ou leurs sous-unités.

Les lectines cerebelleuses solubles ou CSL sont décrites par ZANETTA et al dans Journal of Neurochemistry, 1987, p.1250-1257. Dans cet article, on rapporte l'isolement à partir du cervelet de rat de CSL et de sous-unités protéiques. Ces CSL et leurs sous-unités précurseurs sont caractérisés par leur affinité pour les glycanes riches en mannose portés par des glycoprotéines insolubles dans des détergents neutres tels que le Triton® X-100.

Les sous-unités telles qu'obtenues par extraction à partir de cervelet de rat selon cet article présentent des poids moléculaires (PM) de 31,5 kDa, 33 kDa et 45 kDa.

L'utilisation définie ci-dessus a conduit à l'élaboration d'une méthode de diagnostic de sclérose en plaques et autre neuropathie démyélinisante caractérisée par
- l'incubation d'un prélèvement d'un patient, notamment de liquide céphalo-rachidien, de sang ou d'un liquide plasmatique tel que le plasma ou le sérum avec une lectine endogène telle que définie ci-dessus, dans des conditions permettant la réalisation d'une réaction entre ladite lectine et des anticorps présents dans les liquides cephalorachidiens et le sang de malades atteints de neuropathies démyélinisantes.
- la révélation du complexe immunologique formé selon les méthodes classiques de révélation des immunoglobulines.

Ce test selon l'invention présente en outre l'avantage de relever de l'analyse immunologique biomédicale classique et de présenter en conséquence un prix de revient modéré.

Les résultats obtenus en appliquant ce test sur un ensemble de prélèvements de liquides céphalo-rachidiens ont montré, comme il ressort des exemples donnés ci-après, sa grande sensibilité ainsi qu'une remarquable spécificité permettant un dépistage précoce de la maladie.

De même, on a constaté chez les patients ayant un diagnostic clinique de sclérose en plaques, et qui possèdent pour la plupart des anticorps anti-CSL dans leur liquide céphalo-rachidien, la présence d'anticorps anti-CSL dans leur sang ou les liquides plasmatiques tel que plasma et sérum.

Ainsi, une dilution de 1 à 1000 par exemple de plasma de patients atteints de sclérose en plaques permet de mettre en évidence de façon très nette la présence d'anticorps anti-CSL.

On mesure l'intérêt de ces résultats qui montrent que la présence de ces anticorps n'est pas proprement spécifique au tissus nerveux et que les lectines cerebelleuses solubles sont une cible privilégiée dans la SEP.

On notera de plus que l'utilisation du sang ou des liquides plasmatiques pour effectuer le test le rend beaucoup plus pratique dans la mesure où il n'implique qu'une prise de sang, beaucoup plus facile à réaliser que la ponction lombaire, nécessitant, elle, une hospitalisation.

En outre, le test peut être pratiqué avec seulement quelques microlitres de sang.

A l'appui du diagnostic effectué sur la base de la méthode de l'invention, il est possible d'effectuer des examens complémentaires en ayant recours par exemple à la RMN (résonnance magnétique nucléaire) qui permet de visualiser des lésions disséminées de la substance blanche.

Les lectines endogènes utilisées dans la méthode de diagnostic de l'invention sont des lectines partiellement purifiées ou des préparations pures.

Les lectines ou leurs sous-unités sont largement distribuées dans les tissus des mammifères, des oiseaux et des poissons et peuvent être isolées à partir de ces tissus selon des techniques de purification variables, mais avantageusement fondées sur l'affinité de ces lectines pour les glycanes riches en mannose.

On utilise des CSL ou de lectines endogènes présentant une parenté immunologique avec les CSL, par exemple des lectines CSL isolées à partir d'autres mammifères, d'oiseaux, de poissons ou d'autres organismes moins évolués.

En variante, la méthode de diagnostic de l'invention est mise en oeuvre avec une ou plusieurs des sous-unités de ces lectines.

Des sous-unités de CSL préférées sont du type de celles obtenues dans le procédé de ZANETTA et al évoqué plus haut.

Il s'agit en particulier des sous-unités de 45 kDa, 33 kDa et 31,5 kDa.

L'incubation des lectines endogènes ou de leurs sous-unités avec le prélèvement à tester est réalisée avantageusement à température ambiante en milieu tampon.

On utilise plus spécialement un milieu tampon de pH voisin de la neutralité de force ionique relativement élevée tel que le tampon de type TBS (10 mM Tris-HCl pH 7,2 contenant 0,15 M de NaCl) ou le tampon de type PBS (25mM NaH₂PO₄ - Na₂HPO₄ pH 7,2 contenant 0,15 M de NaCl).

L'étape de révélation des immunoglobulines fixées sur les lectines endogènes CSL est réalisée avantageusement en utilisant soit des immunoglobulines anti-Ig-humaines marquées ou des anti-IgG marquées, soit des molécules de type non immunitaires.

Les immunoglobulines sont marquées, notamment avec une enzyme ou un réactif radioactif.

Comme enzyme, on citera la peroxydase, la phosphatase alcaline, la ß-galactosidase qui correspondent aux enzymes de marquage les plus utilisés à ce jour.

Un réactif classique est constitué par de l'iode radio-actif. Les molécules de type non immunitaires sont par exemple constituées par la protéine A marquée. Le marquage radioactif est effectué le plus généralement par l'iode. Il est clair que d'autres enzymes ou réactifs radio-actifs, ou encore d'autres formes de marquage sont utilisables dès lors qu'ils permettent de révéler la réaction antigène-anticorps considérée.

L'invention vise également un kit de diagnostic apte à effectuer le procédé de diagnostic de l'invention. caractérisé en ce qu'il comprend :
- au moins une lectine endogène ou une de ses sous-unités, telles que définies ci-dessus;
- les solvants et agents nécessaires pour l'étape d'incubation et/ou de révélation.

D'autres caractéristiques et avantages de l'invention sont rapportés dans les exemples qui suivent relatifs a l'obtention de CSL partiellement purifiée et à son utilisation pour détecter la présence d'anticorps anti-CSL chez des patients atteints de sclérose en plaques.

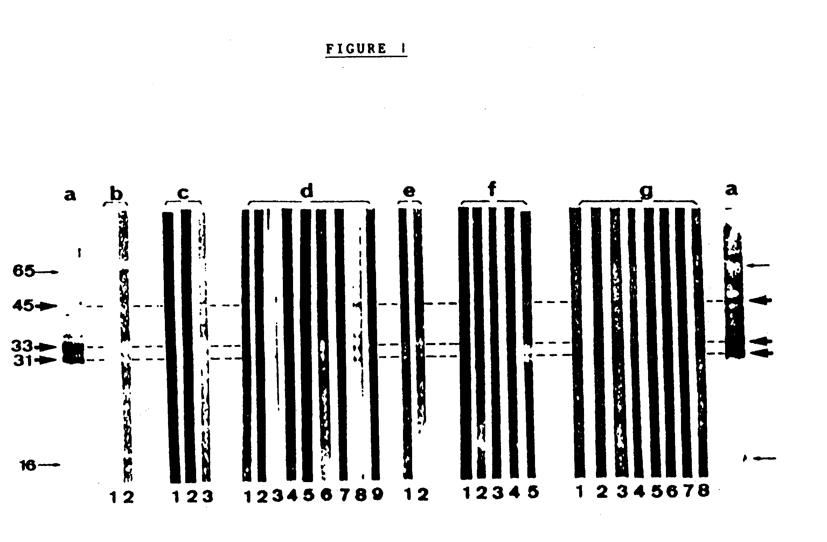

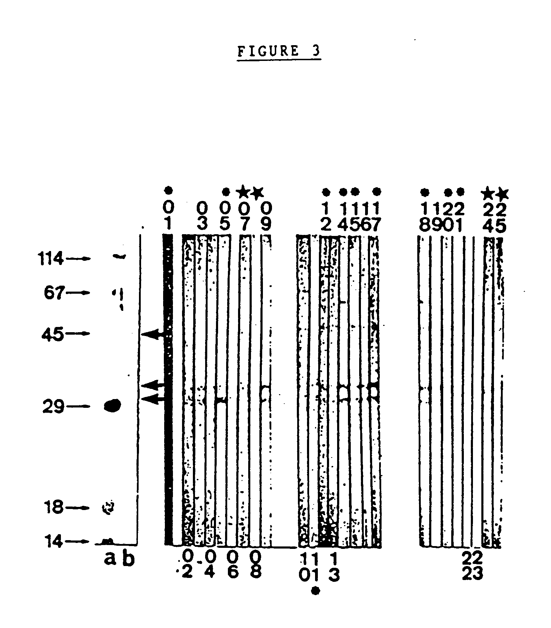

Dans ces exemples, il est fait référence aux figures 1 et 3 qui représentent respectivement :
- les figures 1 et 3 des photos d'immunoblots obtenus avec des prélèvements respectivement de liquides céphalo-rachidiens, et de plasma
- la figure 2, les histogrammes de distribution des diagnostics.

### EXEMPLES

### 1. PREPARATION D'UNE FRACTION ANTIGENIQUE DE CSL PARTIELLELLENT PURIFIEE

On procède à une solubilisation spécifique de CSL avec du mannose, comme décrit dans l'article de ZANETTA et al précité, la CSL provenant de cervelets de jeunes rats. Cette opération et les suivantes sont effectuées à 4°C, en utilisant des tampons auxquels on ajoute, juste avant utilisation, des inhibiteurs de protéase, à savoir du fluorure de phénylméthyl sulfonyle (PMSF) et de l'ester méthylique de la p-tosyl-arginine (respectivement 0,1 et 0,3 mM).

Le matériel solubilisé en présence de mannose purifié (fraction TNM), est précipité à 4°C, durant 30 min., par addition d'acide trichloracétique à 15%, puis on centrifuge à 4°C.

On lave le culot dans du méthanol pur. On sèche sous un léger courant d'air, puis on solubilise à raison de 1mg/ml dans le tampon dissociant de Laemmli. On chauffe ensuite 5 mn à 90°C avant d'effectuer une électrophorèse.

L'électrophorèse est réalisée sur un gel de polyacrylamide (à 13%) en présence de SDS dans le système tampon de Laemmli sur des plaques de gel de 0,75mm d'épaisseur.

On dépose 200µl d'une fraction enrichie en CSL sur une longueur de 130mm et on laisse la migration se développer durant 6 heures sous courant constant de 15mA. Le gel est transferré sur des filtres de nitrocellulose selon les techniques classiques.

Après 4h de transfert sous voltage constant (36V), avec une intensité de 0,4 Amp., le bord du blot est coloré avec de l'Amido Black^{R}. Le reste du filtre de nitrocellulose est saturé avec une solution d'albumine de sérum bovin ou BSA (3%) dans un tampon Tris-HCl (10mM), pH 7,2 contenant 150mM de NaCl (tampon TBS), pendant 2 heures à température ambiante.

Le filtre est lavé à l'eau et conservé dans l'eau à 4°C jusqu'à utilisation.

### 2. INCUBATION DE LA FRACTION ANTIGENIQUE AVEC UN PRELEVEMENT DE LIQUIDE CEPHALO-RACHIDIEN DE PATIENTS ATTEINTS DE MALADIES NEUROLOGIQUES DIVERSES

Le filtre de nitrocellulose précédemment obtenu est découpé en bandes de 2 mm de largeur qui sont transférées sur les pistes d'un récipient d'incubation contenant 0,75 ml d'une solution à 3% de BSA dans le tampon TBS.

Après incubation avec faible agitation pendant au moins 1 heure à température ambiante, on ajoute 0,75 ml de liquide céphalo-rachidien.

Les bandes sont soumises à agitation durant 3 heures à température ambiante, puis sont lavées à 15 reprises, sur une période de 2 heures, incubées 10 min. dans 3% d'BSA dans le tampon du TBS et rincées dan TBS pour une autre période de 2 heures.

### 3. MARQUAGE ET REVELATION DE COMPLEX ANTIGENE-ANTICORPS

On ajoute aux bandes obtenues après rinçage de la BSA (1,5 ml d'une solution à 3% dans TBS) et on incube pendant 30 min. à température ambiante avant addition d'IgG anti-humaines de chèvre marquées avec HRP ou de la phosphatase alcaline (dilution finale 1/500). Les IgG proviennent de la Société IMUNOTECH ou PELFREEZ BIOCHEMICALS.

Les échantillons sont soumis à agitation durant environ 14 heures à 4°C et rincés comme décrit ci-dessus.

La révélation des échantillons est effectuée selon la technique au chloronaphtol pour HRP avec un substrat Promega^{R} pour la phosphatase alcaline.

Après réaction à température ambiante, les bandes sont lavées dans l'eau et séchées entre des filtres de papier.

Les échantillons positifs correspondent à ceux montrant une coloration des bandes ayant des PM de 45, 33 ou 31,5 kDa caractéristiques de CSL. Cette coloration des bandes de CSL n'apparaît pas dans les expériences effectuées en l'absence d'anticorps du liquide céphalo-rachidien ou en utilisant les liquides céphalo-rachidiens de patients ayant d'autres maladies neurologiques que la sclérose en plaques.

### 4. RESULTATS

La photo rapportée sur la figure 1 montre un échantillonage des immunoblots permettant de révéler la présence d'anticorps anti-CSL dans le liquide céphalo-rachidien de patients atteints de sclérose en plaques ou d'autres maladies neurologiques.

Les résultats des figures a) à g) correspondent aux situations suivantes :
a) coloration d'une préparation de protéine utilisée comme antigène à l'aide d'Amido Black^{R} (fraction TNM de cervelets de jeunes rats enrichie en CSL). On constate dans cette préparation partiellement purifiée la présence de bandes à 31,5, 33 et 45 kDa, caractéristiques des sous-unités de la CSL;
b) coloration obtenue en l'absence de liquide céphalo-rachidien et en utilisant des anticorps anti-humains de chèvre marqués avec HRP ou AKP;
c) aspect obtenu en utilisant du liquide céphalo-rachidien de patients présentant des hémorragies méningées dues à des traumas mécaniques. On note une coloration de fond avec absence de bandes colorées définies;
d) et g) correspondent à l'immunodétection de bandes de CSL en utilisant le liquide céphalo-rachidien de patients avec la sclérose en plaques comme sources d'anticorps anti-CSL; la révélation du complexe antigène-anticorps est effectuée en utilisant des immunoglobulines anti-humaines marquées avec HRP ou AKP. On observe que les sous-unités colorées de CSL varient d'un patient à l'autre. La sous-unité de 31,5 kDa est révélée dans d) 1-3, la sous-unité de 45 kDa dans d) 4-9. Dans quelques cas, les sous-unités de 31,5 et 33 kDa de CSL sont fortement colorées avec un schéma de coloration différent de celui de la sous-unité de 45 kDa;
e) et f) correspondent aux mêmes expériences mais avec les liquides céphalo-rachidiens de patients atteints d'autres maladies neurologiques que la sclérose en plaques. e) correspond à des maladies non infectieuses n'affectant pas la barrière hémato-encéphalique et f) à des cas de méningite virale. Dans 4 cas, une bande à 67 kDa a été colorée à la suite d'une réaction immunologique. Chez un patient de la série d'expériences, cette bande n'est observée que durant les phases aigües de la maladie. Des bandes de 40 kDa et 27 kDa sont détectées dans quelques cas.

Ces résultats montrent que les immunoblots de patients atteints de sclérose en plaques diffèrent totalement de ceux des patients ayant d'autres maladies neurologiques. Les bandes ne sont pas colorées lorsqu'on n'ajoute pas de liquide céphalo-rachidien (voir figure 1 b)) ou quand les liquides céphalo-rachidiens proviennent de patients ayant des pathologies différentes de la sclérose en plaques (figure 1c), 1 e) et 1 f)).

255 échantillons de liquides céphalo-rachidiens ont été analysés au cours de ces expériences correspondant à plus de 240 patients différents. Pour les échantillons analysés 2 fois ou plus, les résultats sont toujours reproductibles pour les patients avec des diagnostics de sclérose en plaques.

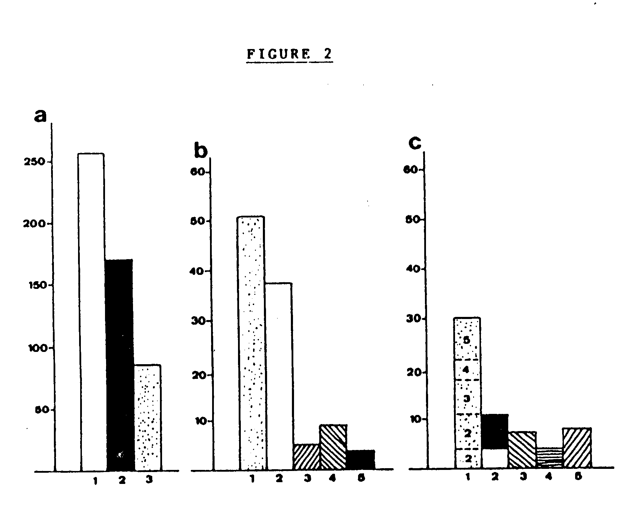

Un résumé de ces résultats est illustré dans la figure 2 qui donne :
a - la distribution d'échantillons donnant des résultats positifs et négatifs à l'analyse par test immunochimique avec CSL comme antigène (1 : échantillons totaux ; 2 : échantillons négatifs ; 3 : échantillons positifs).
b - correspond à la distribution des échantillons de sclérose en plaques diagnostiquées, probables et possibles donnant des tests positifs et des tests négatifs (1 : échantillons totaux positifs ; 2-4 : sclérose en plaques cliniquement diagnostiquée, probable et possible donnant respectivement des tests positifs ; 5 : sclérose en plaques, cliniquement diagnostiquée donnant des tests négatifs.
c - correspond à la distribution d'échantillons au diagnostic différent sans sclérose en plaques donnant des réactions positives dans le test (1 : échantillons totaux ; 2 : polyneuropathies (clair) et syndromes cérébraux et pyramidaux (sombre); 3 : méningite ; 4 : hydrocéphalie ; 5 : autres maladies.

Des résultats négatifs ont été obtenus avec 170 liquides céphalo-rachidiens différents provenant de patients avec des maladies neurologiques variées autres que la sclérose en plaques (démence, Parkinson, syndromes vestibulaires, syndromes pseudo-bulbaires, syndromes extrapyramidaux, douleurs, lombalgies, méningites, hémorragies, etc ...).

Un lot d'échantillons correspondant à des patients avec des méningites hémorragiques montre une coloration de fond et une coloration spécifique d'une bande à 67 kDa non reliée à des bandes de CSL.

Pour 51 cas de sclérose en plaques (y compris la sclérose en plaques diagnostiquée cliniquement, la sclérose en plaques probable et la sclérose en plaques possible), on trouve une coloration des bandes de CSL dans 47 cas. Des résultats négatifs sont obtenus dans seulement 4 cas. Ces 4 cas correspondent à des scléroses en plaques cliniquement diagnostiquées. Dans 30 cas, une coloration des bandes de CSL est détectée sans diagnostic de sclérose en plaques. Ces cas correspondent à un faible nombre de maladies incluant les polyneuropathies (4 cas), les syndromes cérébraux et pyramidaux (6 cas), les atrophies olivo-ponto-cérébellaire (1 cas); méningites (7 cas), les hydrocéphalies (4 cas) et les autres maladies individuelles (septicémie, BROWN-SEQUARD C8D1, tumeur et épilepsie, dysesthesie, leuco-encéphalite postradique, PF périphérique). Ces cas vont faire l'objet d'un examen spécifique en IRM.

La spécificité du test défini comme le rapport des échantillons négatifs vrais réels à la somme des échantillons négatifs réels et des faux positifs, on obtient une valeur minimale de 85% ce qui montre la grande spécificité du test.

Les valeurs concernant la sensibilité sont de l'ordre de 93,5% (rapport des échantillons positifs réels à la somme des échantillons réels et des faux négatifs).

Ces résultats montrent que la mise en évidence de la présence d'anticorps anti-CSL constitue un bon critère d'indication d'un diagnostic de sclérose en plaques.

### 5. UTILISATION DE PLASMAS

Les plasmas, obtenus après centrifugation du sang prélevé sur héparine, sont congelés et gardés à -80°C jusqu'au moment du test.

On rapporte sur la figure 3 les résultats du test par la technique d'immunoréplique de la présence d'anticorps anti-CSL sur une série d'échantillons de plasma de patients atteints de diverses maladies neurologiques.

a) et b) indiquent la coloration par le noir amido : a) des marqueurs de masse moléculaire (masse exprimée en kDa) et b) des protéines utilisées pour le test (les pointes de flèche indiquent la position des sous-unités caractéristiques de la CSL (31,5-33 et 45 kDa).

Les échantillons ayant donné des résultats positifs sont numérotés en haut, les autres en bas. Les échantillons marqués d'un astérisque sont ceux pour lesquels le diagnostic de sclérose en plaques a été formulé.

On note une forte intensité de la coloration des bandes à 31,5 et 33 kDa pour la plupart des patients atteints de SEP. Un résultat négatif (dans le plasma comme pour le LCR) a été obtenu pour l'échantillon N° 11 qui ne présente pas d'images de plaques par imagerie de résonnance magnétique nucléaire. Les échantillons marqués d'une étoile correspondent à un patient atteint de polyradiculonévrite dont le plasma a été prélevé avant (07 et 24) et après (08 et 25) plasmaphorèse. A faible concentration de plasma (1,5 µl pour le test), on observe une diminution des anticorps anti-CSL après plasmaphorèse, mais ils restent détectables à plus forte concentration (15 µl de plasma pour les échantillons 24 et 25). L'échantillon N° 21 correspond à une SEP à synthèse intrathécale d'IgA, ayant donné un premier résultat négatif dans le LCR après révélation de l'anticorps fixé par des anti-IgG. L'utilisation d'anti-Ig au lieu d'anti-IgG permet donc de révéler ce cas positif.

## Revendications

1. Utilisation pour le diagnostic in vitro de neuropathies démyélinisantes, notamment de la sclérose en plaques ou SEP de lectines endogènes ayant une affinité pour les glycanes riches en mannose, ou de leurs sous-unités protéiques, ces lectines, ou leurs sous-unités, présentant une parenté immunologique respectivement avec les lectines cerebelleuses solubles (cerebellar soluble lectin, CSL) ou leurs sous-unités protéiques.

2. Méthode de diagnostic de sclérose en plaques et autres neuropathies démyélinisantes, caractérisée par
- la mise en contact d'un prélèvement d'un patient notamment du liquide céphalo-rachidien ou du sang ou des liquides plasmatiques tels que le sérum ou le plasma, avec une lectine endogène telle que définie selon la revendication 1 dans des conditions permettant la réalisation d'une réaction immunologique entre ladite lectine et des anticorps présents dans les liquides cephalorachidiens et le sang de malades atteints de neuropathies démyélinisantes.
- la révélation du complexe immunologique éventuellement formé selon les méthodes classiques de révélation des immunoglobulines.

3. Méthode selon la revendication 2, caractérisée en ce qu'on met en oeuvre une préparation de lectines totalement ou partiellement purifiées.

4. Méthode selon la revendication 2 ou 3, caractérisé en ce qu'on utilise une ou plusieurs sous-unités protéiques des lectines.

5. Méthode selon l'une quelconque des revendications 1 à 4, caractérisé en ce qu'on utilise de la CSL ou une ou plusieurs sous-unités de CSL, en particulier celles de PM de 45 kDa, 33 kDa ou 31,5 kDa.

6. Méthode selon l'une quelconque des revendications 1 à 5, caractérisée en ce que l'étape de mise en contact est réalisée par incubation à température ambiante en milieu tampon.

7. Méthode selon l'une quelconque des revendications 1 à 6, caractérisée en ce que l'étape de révélation des immunoglobulines fixées sur les lectines endogènes CSL est réalisée avantageusement en utilisant soit des immunoglobulines anti-Ig-humaines marquées ou anti-IgG marquées, soit des molécules de type non immunitaires.

8. Kit de diagnostic de sclérose en plaques et plus généralement de neuropathies démyélinisantes, permettant la mise en oeuvre du procédé selon l'une quelconque des revendications 2 à 7, caractérisé en ce qu'il comporte :
- au moins une lectine endogène du type CSL ou une de ses sous-unités, telles que définies dans la revendication 1.
- les solvants et agents nécessaires pour l'étape d'incubation et/ou de révélation.

## Patentansprüche

1. Verwendung von endogenen Lectinen, die eine Affinität für die Mannöse-reichen Glycane besitzen, oder von deren eiweißartigen Untereinheiten zur in vitro-Diagnose von entmyelinisierenden Neuropathien, vor allem von multipler Sklerose oder M.S. wobei diese Lectine oder ihre Untereinheiten eine immunologische Verwandtschaft mit den zerebellar-löslichen Lectinen (cerebellar soluble lectine, CSL) oder deren eiweißartigen Untereinheiten aufweisen.

2. Diagnoseverfahren für multiple Sklerose und andere entmyelinisierende Neuropathien, dadurch gekennzeichnet, daß man
- eine Probe eines Patienten, vor allem kephalo-rachiale Flüssigkeit oder Blut oder plasmatische Flüssigkeiten, wie Serum oder Plasma, mit einem endogenen Lectin, wie in Anspruch 1 definiert, unter Bedingungen in Berührung bringt, die den Ablauf einer immunologischen Reaktion zwischen dem Lectin und in den kephalo-rachialen Flüssigkeiten und im Blut von Kranken, die an entmyelinisierenden Neuropathien leiden, enthaltenen Antikörpern ermöglichen und
- den gegebenenfalls gebildeten immunologischen Komplex mit Hilfe klassischer Nachweisverfahren für Immunoglobuline nachweist.

3. Verfahren nach Anspruch 2, dadurch gekennzeichnet, daß man ein Präparat von vollständig oder partiell gereinigten Lectinen einsetzt.

4. Verfahren nach Anspruch 2 oder 3, dadurch gekennzeichnet, daß man eine oder mehrere eiweißartige Untereinheiten der Lectine verwendet.

5. Verfahren nach einem der Ansprüche 1 bis 4, dadurch gekennzeichnet, daß man CSL oder eine oder mehrere Untereinheiten von CSL, insbesondere solche mit einem Molekulargewicht von 45 kDa, 33 kDa oder 31,5 kDa verwendet.

6. Verfahren nach einem der Ansprüche 1 bis 5, dadurch gekennzeichnet, daß man die Stufe des Inberührungbringens durch Inkubieren bei Raumtemperatur in einem Puffermedium realisiert.

7. Verfahren nach einem der Ansprüche 1 bis 6, dadurch gekennzeichnet, daß man die Stufe des Nachweises der auf den endogenen CSL-Lectinen fixierten Immunoglobulinen vorteilhafterweise realisiert, indem man entweder markierte anti- menschliche-Ig Immunoglobuline oder markierte Anti-IgG oder Moleküle vom nicht-immunitären Typ verwendet.

8. Diagnosekit für multiple Sklerose und allgemein für entmyclinisierende Neuropathien zur Durchführung des Verfahrens nach einem der Ansprüche 2 bis 7, dadurch gekennzeichnet, daß er enthält:
- mindestens ein endogenes Lectin vom CSL-Typ oder eine seiner Untereinheiten, wie in Anspruch 1 definiert, und
- die Lösungsmittel und notwendigen Mittel für die Stufe der Inkubation und/oder des Nachweises.

## Claims

1. Use for the in vitro diagnosis of demyelinating neuropathies, in particular of multiple sclerosis or MS, of endogenous lectins which have an affinity for glycans rich in mannose, or their protein subunits, these lectins or their subunits exhibiting an immunological relationship with the cerebellar soluble lectins (CSL) or their protein subunits, respectively.

2. Diagnostic method for multiple sclerosis and other demyelination neuropathies, characterized by :
- the placing of a sample taken from a patient, in particular cerebrospinal fluid or blood or plasma fluids such as serum or plasma, in contact with an endogenous lectin such as defined according to Claim 1 under conditions enabling an immunological reaction to occur between the said lectin and antibodies present in the cerebrospinal fluid and the blood of patients affected by demyelinating neuropathies,
- the revelation of the immunological complex possibly formed, according to the standard procedures for the revelation of immunoglobulins.

3. Method according to Claim 2, characterized in that a preparation of completely or partially purified lectins is used.

4. Method according to Claim 2 or 3, characterized in that one or more protein subunit(s) of the lectins is/are used.

5. Method according to any one of the Claims 1 to 4, characterized in that the CSL or one or more subunits of CSL is/are used, in particular those of MW of 45 kDA, 33 kDa or 31.5 kDa.

6. Method acording to any one of the Claims 1 to 5, characterized in that the step involving the placing in contact is carried out by incubation at room temperature in a buffer.

7. Method according to any one of the Claims 1 to 6, characterized in that the step involving the revelation of the immunoglobulins bound to the endogenous lectins CSL is advantageously carried out by using either labelled anti-IgG or labelled anti-human Ig immunoglobulins, or molecules of non-immune types.

8. Diagnostic kit for multiple sclerosis, and more generally for demyelinating neuropathies,permitting the implementation of the procedure according to any one of the Claims 2 to 7, characterized in that it contains :
- at least one endogenous lectin of the CSL type or one of its subunits, such as defined in Claim 1;
- the solvents and reagents necessary for the incubation and/or revelation step.
